Europäisches Patentamt

European Patent Office

Office européen des brevets

(19)

(11) Numéro de publication: **0 343 080**
**A1**

(12)

# DEMANDE DE BREVET EUROPEEN

(21) Numéro de dépôt: **89401381.2**

(22) Date de dépôt: **19.05.89**

(51) Int. Cl.⁴: **C 12 N 1/04**
G 01 N 33/569,
G 01 N 33/571, C 12 N 5/00

(30) Priorité: **19.05.88 FR 8806698**

(43) Date de publication de la demande:
**23.11.89 Bulletin 89/47**

(84) Etats contractants désignés:
**AT BE CH DE ES FR GB GR IT LI LU NL SE**

(71) Demandeur: **IRE-CELLTARG S.A.**
**Zone Industrielle**
**B-6220 Fleurus (BE)**

(72) Inventeur: **Pirson, Philippe Jean Achille Louis**
**25, avenue de Burbure**
**B-1970 Wezmbeck-Oppem (BE)**

(74) Mandataire: **Warcoin, Jacques et al**
**Cabinet Régimbeau 26, avenue Kléber**
**F-75116 Paris (FR)**

(54) **Test diagnostique de maladie virale et procédé de congélation de cellules utiles pour ledit test.**

(57) Test diagnostique de maladie virale et procédé de congélation de cellules utiles pour ce test.

La présente invention a pour objet un test diagnostique de maladie virale comportant l'utilisation de cellules permettant d'amplifier le virus à identifier à partir de prélèvements et rendant ainsi sa détection possible, caractérisé en ce qu'on utilise des cellules congelées que l'on décongèle pour procéder au test.

La présente invention a également pour objet un procédé de congélation de cellules dans un récipient support consistant notamment dans des plaques multipuits ou des tubes permettant la conservation de cellules et leur utilisation après décongélation dans un test de diagnostique selon l'invention, caractérisé en ce que

1) on utilise un cryopréservant choisi de sorte que le nombre de cellules viables et fonctionnelles après décongélation soit optimal et notamment supérieur à 80%, et

2) le nombre de cellules à congeler introduites dans le récipient support est choisi de manière à ce que 24 heures après décongélation les cellules cultivées forment une monocouche cellulaire complète.

**Description**

## TEST DIAGNOSTIQUE DE MALADIE VIRALE ET PROCEDE DE CONGELATION DE CELLULES UTILES POUR LEDIT TEST.

La présente invention concerne un test diagnostique de maladie virale ainsi qu'un procédé de congélation de cellules utile pour conserver des cellules en vue dudit test diagnostique.

Le diagnostic de maladies infectieuses et l'identification de l'agent responsable nécessitent, à l'heure actuelle, une réponse rapide et précise, afin de permettre le traitement de la maladie avec le médicament approprié ou la décision d'un acte médical particulier. Dans le cas des maladies virales, un diagnostic rapide est rendu nécessaire pour les virus vis-à-vis desquels un traitement est actuellement possible (virus Herpes simplex ou HSV) ou pour lesquels une identification précise est importante (cystomegalovirus, virus syncytiel respiratoire...).

Le diagnostic des virus au laboratoire repose sur deux méthodes d'une part, la détection directe des antigènes spécifiques, d'autre part, la culture du virus sur des souches de cellules réceptives.

La première méthode implique l'utilisation d'anticorps (monoclonaux ou polyclonaux) reconnaissant un ou plusieurs antigènes viraux caractéristiques de l'espèce, et détecté par immunofluorescence, immunoperoxidase, ELISA et plus récemment de sonde à ADN ou ARN. L'avantage de cette méthode est l'identification spécifique du virus (antigène), des particules virales vivantes n'étant pas requises. Les inconvénients majeures de cette méthode sont le manque de sensibilité, la nécessité d'échantillon de bonne qualité et les variables techniques permettant de distinguer un spécimen positif d'un échantillon négatif (Schmidt etal, 1983, J. Clin. Microbiol. 18 : 445-448; Nerurkar etal, 1984, J. Clin.Microbiol., 19 : 631:633).

La seconde méthode de diagnostic des virus utilise des souches de cellules maintenues en culture de façon continue dans les laboratoires. Ces cellules permettent d'amplifier le virus à identifier, à partir de prélèvements, et de rendre sa détection possible après 24 heures par l'observation de plage de cytolyse, l'identification d'antigènes viraux par des anticorps spécifiques ou l'utilisation de sonde ADN. Cette méthode reste la technique de référence par sa grande sensibilité et l'amplification de matériel vivant nécessaire pour un diagnostic précis.

Toutefois la culture des cellules en continu requise par ces techniques de diagnostiC constitue un inconvénient majeur dans leur utilisation.

C'est pourquoi la présente invention propose une méthode de congélation-décongélation de cellules permettant leur utilisation pour le diagnostiC viral dans les 24 heures.

La présente invention a en effet pour objet un test diagnostique de maladie virale comportant l'utilisation de cellules permettant d'amplifier le virus à identifier, à partir de prélèvements et rendant sa détection possible après culture, caractérisé en ce qu'on utilise des cellules congelées que l'on décongèle pour le test.

Selon les tests de diagnostiC selon l'invention la détection du virus peut se faire par l'iden tification d'antigènes viraux par des anticorps spécifiques, l'utilisation de sondes ADN ou plus simplement l'observation de plage de cytolyse.

On peut citer à titre purement illustratif et non limitatif les tests qui permettent de détecter le virus Herpes simplex, le cytomegalovirus, ou le virus syncytiel respiratoire.

Selon l'invention l'échantillon de virus infectueux peut être mélangé avec les cellules dès après leur décongélation, l'agent viral pouvant être identifié après 24 heures d'incubation par une méthode spécifique (antigène, ADN).

La présente invention a donc également pour objet un procédé de congélation de cellules dans un récipient support consistant notamment dans des plaques multipuits ou des tubes permettant l'utilisation des cellules après décongélation, notamment dans un test de diagnostiC selon l'invention. La mise au point des méthodes de congélation-décongélation selon l'invention pose certaines difficultés dans la mesure où il est nécessaire de maintenir après décongélation outre une viabilité des cellules optimale, également une sensibilité à l'agent viral.

Selon la caractéristique essentielle de ce procédé,
- d'une part on utilise un cryopréservant choisi de manière à ce que le nombre de cellules viables et fonctionnelles, c'est-à-dire gardant une sensibilité à l'agent viral, après décongélation soit optimal et notamment supérieur à 80 %.
- d'autre part le nombre de cellules à congeler introduites dans le récipient support est choisi de manière à ce que les cellules cultivées forment une monocouche cellulaire complète 24 heures après décongélation.

Ainsi se trouvent réunies les meilleures conditions pour procéder au test de diagnostiC viral, notamment lorsque la détection se fait par l'observation de plage de cytolyse.

Avantageusement le récipient-support est en matière plastique traitée préalablement avec un agent de revêtement choisi notamment parmi le collagène, la fibronectine, la polylysine, la concanavaline et des mélanges de ceux-ci, en particulier les supports sont traités par un mélange de collagène et fibronectine.

Les supports ainsi traités permettent d'obtenir :
-d'une part un bon étalement, c'est-à-dire un étalement homogène des cellules et
-d'autre part un meilleur attachement des cellules sur le support,
qui sont également les conditions idéales pour procéder au test de diagnostique.

L'agent cryopréservant selon l'invention consiste en un agent cryoprotecteur dilué.

On peut citer comme agent cryoprotecteur selon l'invention le DMSO, le glycérol, le tréhalose, le diméthylacétamide, le polyéthylèneglycérol, le polyvinyl pyrolidone.

Le diluant peut être le milieu de culture seul, de préférence le milieu additionné de sérum de veau foetal (SFV) notamment 5 à 10 %, ou de sérum albumine de bovin notamment 2 à 5 %, ou le SFV pur.

On peut effectuer la congélation de cellules en suspension dans un récipient pouvant être le récipient support ou en culture monocouche dans le récipient support.

On peut citer comme cellules à titre illustratif :

. les cellules embryonnaires de poumon humain de type fibroplastique MRC-5, et

. les cellules de rein de singe de type fibroplastique VERO.

L'utilisation du procédé de congélation selon la présente invention est particulièrement intéressante en vue de l'amplification des virus pour leur identification mais cette utilisation n'est pas limitative.

D'autres avantages et caractéristiques de l'invention apparaîtront à la lumière des exemples suivants.

## Exemple 1 : LA CULTURE DES CELLULES

La lignée cellulaire MRC-5 (cellules embryonnaires de poumon humain de type fibroblastique) et la lignée VERO (cellules de rein de singe de type fibroblastique) ont été choisies à titre d'exemple. Ces deux lignées sont sensibles aux virus Herpes, Cytomégalovirus et virus syncytiel respiratoire et sont actuellement les plus utilisées. D'autres lignées cellulaires peuvent être envisagées pour le développement d'autres souches virales.

Les cellules sont cultivées à 37°C dans une atmosphère saturée en eau sous le rapport $CO_2$/air (7:93, v/v) dans des flacons de culture tissulaire en plastique de 175 cm$^2$ (T175). Le milieu consiste en du RPMI-1640 contenant 10 mM de $NaHCO_3$, 20 mM de Hepes, 2 mM de glutamine, 50μg/ml de streptomycine, 50 U/ml de penicilline et additionné de 1% d'une solution d'acides aminés non essentiels et de 10 % de sérum de foetus de veau inactivé par la chaleur (SFV). Le milieu doit être renouvelé 2 fois par semaine. Les cultures sont repiquées dès confluence cellulaire tous les 5 à 7 jours selon le type cellulaire. Les cultures repiquées sont préparées par trypsinisation des monocouches de cellules, avec un mélange trypsine-EDTA comprenant 0,05 % (poids/volume) de trypsine et 0,02 % (poids/volume) d'EDTA. Pour ce faire, le vieux milieu est enlevé, les cellules lavées avec une solution saline tamponnée et incubées quelques minutes à 37°C avec la solution trypsine-EDTA. Les cellules sont remises en suspension dans le milieu de culture et transférées dans de nouvelles boîtes avec un rapport de repiquage de 1 : 5.

## Exemple 2 : PARAMETRES D'EVALUATION ET SENSIBILITE A L'INFECTION VIRALE .

1) Différents critères ont été étudiés afin d'évaluer les cellules en culture après le processus de congélation et décongélation. Les paramètres utilisés sont les suivants :

La viabilité cellulaire, après décongélation, est déterminée par le test d'exclusion du trypan bleu (solution à 0,5 % en poids dans une solution saline tampon de phosphate) et par le test du sel de tétrazolium (MTT) selon la technique décrite par Gerlier et Thomasset (J. Immunol. Methods, 1986, 94 : 57-63). Ce test évalue la viabilité cellulaire sur base de la fonctionnalité biochimique des cellules. Le composé, soluble et incolore dans le milieu de culture, est transformé par des enzymes mitochondrieux (déhydrogenases) en cristaux de formazan colorés et insolubles. Le précipité est solubilisé dans une solution d'isopropanol contenant 0,04 N d'HCl et sa concentration est déterminée à l'aide d'un spectrophotomètre aux longueurs d'ondes de 570 nm et 630 nm. Une lecture automatique peut être réalisée dans un lecteur de type ELISA pour plaque multipuits. Une régression linéaire entre la concentration de produit transformé et le nombre de cellules est établie pour chacune des lignées cellulaires et permet ainsi d'évaluer le nombre de cellules viables (fonctionnelles) de chacune des préparations étudiées.

Le nombre de cellules totales est déterminé soit par comptage des cellules dans un hémocytomètre après coloration des noyaux au bleu de Turck non dilué, soit après fixation des cellules en culture et coloration des noyaux à l'haematoxyline. Pour ce faire, les cellules sont fixées à l'aide d'une solution d'acide acétique glacial-méthanol (1:3, v:v) à raison d'un volume de fixateur par volume de milieu de culture. Après 5 minutes, le milieu est aspiré et les cellules sont lavées 2 x 2 minutes avec le même fixateur. Après rinçage avec l'eau pour enlever le fixateur restant, les cellules sont séchées à l'air puis colorées à l'haematoxyline (solution commerciale) pendant 5 minutes. Les cellules sont ensuite rincées à l'eau de robinet 2 à 3 fois puis séchées à nouveau.

Ce test permet une lecture automatique à l'aide d'un lecteur ELISA à la longueur d'onde de 570 nm. Une régression linéaire est également établie entre l'absorbance mesurée et le nombre total de cellules, et ce, pour chacune des deux lignées cellulaires.

L'observation microscopique au contraste de phase permet de définir l'aspect morphologique des cellules, l'étalement cellulaire et la répartition des cellules sur le support (homogène ou non), ainsi que l'aspect de la monocouche cellulaire à confluence des cellules. Le délai nécessaire pour obtenir une parfaite couche cellulaire confluente est également déterminé par l'observation microscopique.

2) Les méthodes apportées par l'invention ont pour but de permettre la conservation de cellules, utilisées pour le diagnostiC de maladie virale, afin de permettre, le moment opportun, de réaliser le test diagnostique.

Il est donc important de déterminer si les cellules ayant subi le processus de congélation + décongélation, ont conservé leur sensibilité à l'agent viral et à quel degré.

Nous avons considéré le virus Herpes simplex de type I (HSV-1) et de type II (HSV-2). Les deux souches virales proviennent de l'ATCC sous le code numéro VR-733 (HSV-1) et VR-734 (HSV-2). Les virus sont cultivés sur cellules HEp 2 dans du milieu MEM additionné de 10 % SFV et sont conservés à -70°C à raison de $2,3.10^8$ et $7.10^7$ particules virales par ml de milieu de culture complet, respectivement pour le HSV-1 et HSV-2.

Un test diagnostique consiste à infecter les cellules (MRC-5 et Vero) confluentes dans des plaques à 24 puits avec une dilution croissante (de 10 X en 10 X) de l'inoculum viral, dans le milieu de culture. A cet effet, le milieu de culture est aspiré et remplacé par la suspension virale à raison de 100 µl par puits (volume minimal couvrant les cellules sans avoir un dessèchement ultérieur). Les cellules sont incubées à 37°C pendant 1 heure. Du milieu frais est ensuite ajouté à chacun des puits et les cellules sont incubées à 37°C. La formation de plages de cytolyse, caractéristiques pour les deux souches d'Herpes, sont observées au microscope à contraste de phase après 24 et 48 heures.

Ce test est réalisé pour les cellules en culture et, les cellules congelées et décongelées selon les méthodes développées ci-après.

Exemple 3 : CONGELATION DE CELLULES EN CULTURE MONOCOUCHE

3.1 Conditions de culture

Les cellules MRC-5 et Vero, cultivées dans le milieu de culture décrit précédemment, sont placées dans des plaques à 24 puits (Linbro, Flow Laboratories) ou des tubes Gencell (GENUS DIAGNOSTICS) à raison de $5 \times 10^4$ cellules par puits ou par tube. Les cellules sont maintenues à 37° jusqu'à l'obtention d'une monocouche complète (après 24 à 48 heures).

3.2 Méthode de congélation et décongélation

Les différents facteurs, cités précédemment, ont été étudiés pour chaque type cellulaire et condition.

Les meilleures conditions définies pour la congélation-décongélation des cellules MRC-5 en culture monocouche en tubes Gencell sont les suivantes. A confluence des cellules, le cryopréservant, une solution à 10 % de tréhalose dans du sérum de veau foetal, est appliqué sur les cellules. Le tube est maintenu 10 minutes à 20° avant d'être congelé directement à -70° (-5°C/min). Les tubes sont conservés à cette température. A la décongélation, le tube est maintenu à 20° jusqu'à dissolution du cryopréservant. Celui-ci est prélevé et remplacé délicatement par du milieu de culture complet à température ambiante. Les cellules sont maintenues à cette température 10 à 15 minutes avant d'être incubées à 37° avec 7 % de $CO_2$ dans l'air. Le milieu de culture est renouvelé après 24 heures.

La même procédure est applicable aux cellules MRC-5 et cellules Vero cultivées en plaques 24 puits, le cryopréservant étant, dans le cas des cellules Vero, du DMSO à 7,5 % dans du SFV.

Les supports cellulaires sont traités avant culture avec du collagène et de la fibronectine. Cette couverture est obtenue en incubant les boîtes ou les tubes pendant 24 heures à 37° avec 1 ml de collagène (100 µg/ml) et de fibronectine (50 µg/ml) dans une solution tampon de phosphate et en les lavant postérieurement avec le même tampon. Les supports sont utilisés tels quels ou sont séchés préalablement à 20°.

3.3 Résultats

L'effet de 3 cryopréservants sur la viabilité cellulaire et l'attachement des cellules MRC-5 du support de tube Gencell est décrit dans le Tableau 1. Le DMSO à 7,5 % et le glycérol à 5% dans du SFV ne permettent pas de maintenir les cellules attachées au support. Le glycérol à 10 % permet de maintenir 37 % des cellules attachées au support après 24 heures, mais la répartition cellulaire est hétérogène et la croissance plus lente que les cellules en culture normale. Le tréhalose à 10 % dans du SFV donne les meilleurs résultats avec 62 % des cellules attachées après 24 heures et une bonne viabilité. Les cellules sont réparties de façon homogène et leur croissance est plus rapide.

Dans le Tableau 2, il apparaît que 75 % (± 13 %) de la surface disponible (tube Gencell) est occupée par les cellules MRC-5, 24 heures après décongélation lorsque le tréhalose à 10 % dans du SFV est utilisé comme cryopréservant. Après 48 heures, les cellules n'occupent plus que 45 % (± 34 %) de la surface. Les résultats montrent un détachement cellulaire prononcé entre 24 et 48 heures et une variation importante est également observée de série à série. Les cellules se multiplient 48 heures après décongélation et nécessitent 6 à 9 jours avant de reformer un tapis cellulaire confluent et ne sont pas utilisables avant 48 heures.

Pour améliorer l'attachement cellulaire, les cellules sont cultivées sur des supports traités au collagène (100 µg) et à la fibronectine (100 µg). La polylysine et la concanavaline A empêchent l'étalement et la croissance des cellules.

Dans ces conditions (tableau 2), le détachement cellulaire après 48 heures est considérablement réduit avec une plus grande homogénéité dans chaque série. La croissance cellulaire est plus rapide, atteignant la confluence en 3 à 5 jours. Les cellules sont utilisables pour des tests de diagnostique après 48 heures de culture.

Il est à remarquer que le traitement du support, améliore encore davantage l'effet du glycérol à 10 % dans du SFV.

Actuellement, les surfaces sont traitées avec un mélange de collagène et de fibronectine à la concentration respective de 100 µg et 50 µg.

Lors de la congélation de cellules en culture monocouche dans des plaques à 24 puits, des résultats similaires sont obtenus à la décongélation (Tableau 3).

4

Tableau 1

Etude de la viabilité des cellules MRC-5 en monocouche et de l'attachement cellulaire après décongélation en fonction des cryopréservants.

| % cryopréserv. dans du SFV | | Viabilité (%)[a] | | Couche Cell.[b] (à 24 h) | Répartition cellulaire | Croissance cellulaire |
|---|---|---|---|---|---|---|
| | | 2 h | 24 h | | | |
| DMSO | 7,5 % | 50 | - | détachement - | | - |
| Glycérol | 5 % | 50 | - | détachement - | | - |
| | 10 % | 70 | 80 | 37 % (30) | hétérogène | lente |
| | 15 % | 50 | 80 | 13 % (24) | hétérogène | lente |
| Tréhalose | 5 % | 70 | 75 | 50 % (20) | ± homogène | ± normale |
| | 10 % | 75 | 85 | 62 % (27) | ± homogène | ± normale |

[a] La viabilité cellulaire est évaluée 2 h et 24 h après la décongélation et est exprimée en % du nombre de cellules présentes à 2 h et 24 h dans les tubes Gencell.
[b] Surface occupée par les cellules 24 h après la décongélation en % de la surface totale (moyenne ± standard; n = 4 séries correspondant à 14 tubes) et calculée à partir du nombre total des cellules présentes à 24 h et à confluence (cultures contrôles).

EP 0 343 080 A1

Tableau 2

Influence du traitement du support des tubes Gencell sur l'attachement des cellules aprés décongélation des cellules MRC-5 en monocouche.

Congélation sur support "traité"

| Cryopréservant | Support[a] | | |
|---|---|---|---|
| | Contrôle | Collagène | Fibronectine |
| 10 % tréhalose | | | |
| 24 h | 75 % (13) | 94 % ( 9) | 77 % (8) |
| 48 h | 45 % (34) | 88 % (13) | 75 % (5) |
| confluence[b] | 6 - 9 | 3 - 5 | 4 - 6 |
| 10 % glycérol | | | |
| 24 h | 62 % (14) | 90 % ( 8) | 68 % (9) |
| 48 h | 20 % ( 0) | 75 % (17) | 66 % (4) |
| confluence[b] | 11 | 3 - 6 | 4 - 7 |

[a] Les résultats représentent la surface occupeé par les cellules 24 et 48 h après la décongélation (n = 2 séries correspondant à 8 tubes).
[b] Représente le délai en jours nécessaire pour obtenir une monocouche de cellules confluentes.

Exemple 4 : CONGELATION DE CELLULES EN SUSPENSION DANS DES PLAQUES MULTIPUITS

4.1 Méthodes

Les cellules MRC-5 et Vero sont détachées des boîtes de culture à l'aide de la solution de trypsine-EDTA. Après détachement, les cellules sont resuspendues dans du milieu complet, centrifugées puis resuspendues dans le même milieu. Après comptage, les cellules sont centrifugées une nouvelle fois puis resuspendues dans le cryopréservant, une solution à 7,5 % de DMSO dans du SFV, à une concentration cellulaire finale de $2,4 \times 10^5$ cellules MRC-5/ml et $4,4 \times 10^5$ cellules Vero/ml.

Les cellules en suspension sont distribuées dans des plaques à 24 puits (Linbro, Flow Laboratory) à raison de 0,5 ml/puits puis congelées directement à -70°C. Les puits de chaque plaque sont préalablement traités avec une mixture de collagène et fibronectine comme décrit à l'exemple 1.2.

A la décongélation, les plaques sont placées à 37° jusqu'à solubilisation complète du cryopréservant. Un volume de 1,5 ml de milieu frais sans sérum est ajouté à chaque puits, et la plaque est centrifugée 5 minutes à 1500 tours. Le milieu est ensuite remplacé par du milieu complet. Les cellules sont resuspendues puis centrifugées à nouveau 5 minutes. Les cellules sont ensuite incubées à 37° sous 7 % de $CO_2$ dans l'air. Le milieu est renouvelé après 24 heures.

Tableau 3

Congélation de cellules MRC-5 en culture monocouche dans des plaques à 24 puits.

| Support[a] | Viabilité (%)[b] | | | Confluence[c] (jours) |
|---|---|---|---|---|
| | décongélation | 24 h | 48 h | |
| Libre | 85 (13) | 42 (23) | 25 ( 8) | - |
| Traité | 95 ( 8) | 85 (12) | 78 (14) | 3 - 6 |

[a] Support libre ou traité avec du collagène (100 µg) et de la fibronectine (50 µg)
[b] La viabilité représente le nombre de cellules viables sur le nombre de cellules totales (à confluence), exprimée en %
[c] Représente le délai en jours, nécessaire pour obtenir une monocouche de cellules confluentes.

EP 0 343 080 A1

### 4.2 Résultats

Dans ces conditions, la viabilité cellulaire varie entre 80 et 85 %. Après décongélation, les cellules sur support traité, occupent au moins 85 % de la surface disponible et forment une monocouche complète après 24 heures de culture. Les cellules sont bien étalées de façon homogène sur le support traité et présentent un aspect morphologique identique aux cellules contrôles. Dans les mêmes conditions, mais sur un support libre, les cellules occupent seulement 65 à 70 % de la surface; la monocouche est formée après 48 à 72 heures. Il est à rappeler que les cellules contrôles (non congelées) forment une couche monocellulaire dans les 24 heures, pour une densité cellulaire initiale de $10^5$ cellules MRC-5/puits et $2\times10^5$ Vero/puits.

Il est à remarquer, que dans ce cas, seul le DMSO comme cryopréservant donne les meilleurs résultats (viabilité et étalement cellulaire).

Les cellules congelées, puis décongelées, sont infectées après 24 heures de culture par des virus Herpes HSV-1 et HSV-2 selon la méthode décrite à l'exemple 2.2). Après 24 heures, les cellules congelées montrent une très bonne sensibilité aux deux souches de virus, néanmoins inférieure d'un facteur 10 X par rapport aux cellules contrôles.

Après 48 heures d'incubation, la sensibilité des cellules congelées rejoint celle des cellules contrôles.

Les cellules sur support traité sont utilisables 24 heures après décongélation mais pourraient l'être directement après décongélation, en mélangeant l'échantillon de virus infectieux avec les cellules après la seconde centrifugation et leur mise en culture. Après 24 heures d'incubation, l'agent viral pourrait être identifié par une méthode spécifique (anti gène, sonde ADN).

### Exemple 5 : CONGELATION DE CELLULES EN SUSPENSION DANS DES TUBES (CRYOTUBES, TUBES DE CULTURE)

#### 5.1 Méthodes

Les cellules, préparées selon la procédure décrite à l'exemple 4.1., sont resuspendues dans une solution DMSO à 7,5 % dans du SFV, à une concentration cellulaire de $5\times10^6$ cellules/ml. Il est à remarquer que la viabilité optimale est obtenue dans le cas des cellules MRC-5 et Vero, à la densité cellulaire respectivement de $2\times10^6$ et $5\times10^6$ cellules/ml de cryopréservant.

Les cellules en suspension sont distribuées dans des tubes, soit des cryotubes de 2 ml de contenance, soit des tubes de culture de 15 ml (Falcon, Becton Dickinson), à raison d'un ml par tube. Les cellules sont ensuite congelées directement à -70°C.

Pour décongeler les cellules, les tubes sont réchauffés à 37° jusqu'à dissolution du cryopréservant. Les cellules sont soit transférées dans un tube contenant 14 ml de milieu frais sans sérum (cryotube), soit diluées directement avec le même volume de milieu (tube de culture). Après centrifugation, les cellules sont resuspendues dans le milieu de culture complet et distribuées dans les puits traités (collagène et fibronectine) à raison de $1,2\times10^5$ cellules MRC-5 et $2,2\times10^5$ cellules Vero par puits.

Les cellules sont incubées à 37° sous 7 % de $CO_2$ dans l'air. Le milieu est renouvelé après 24 heures.

#### 5.2 Résultats

Dans ces conditions, la viabilité cellulaire est voisine de 84 % ($\pm$ 3 %) pour les cellules MRC-5 (n = 15) et de 86 % ($\pm$ 5 %) pour les cellules Vero (n = 8). La viabilité cellulaire est optimale si la densité des cellules MRC-5 est au moins de $2\times10^6$ cellules/ml de cryopréservant et la densité des cellules Vero est égale à $5\times10^6$ cellules/ml de cryopréservant.

A nouveau, l'étalement optimal des cellules est obtenu dans le cas de support traité. La densité de cellules utilisée pour l'ensemencement des cultures, permet d'obtenir une couche monocellulaire confluente, 24 heures après la décongélation.

Cette méthode est simple, reproductible et permet de préserver la sensibilité des cellules MRC-5 et Vero au virus Herpes (HSV-1 et HSV-2). Comme mentionné dans l'exemple 4, il est possible d'ajouter l'échantillon de virus après décongélation, lors de la mise en culture des cellules.

### Revendications

1 - Test diagnostique de maladie virale comportant l'utilisation de cellules permettant d'amplifier le virus à identifier à partir de prélèvements et rendant ainsi sa détection possible, caractérisé en ce qu'on utilise des cellules congelées que l'on décongèle pour effectuer le test.

2 - Test diagnostique selon la revendication 1 caractérisé en ce que la détection du virus se fait par l'observation de plage de cytolyse, l'identification d'antigènes viraux par des anticorps spécifiques ou l'utilisation de sondes ADN.

3 - Test diagnostique selon l'une des revendications précédentes caractérisé en ce que le virus est le virus Herpes simplex, le cytomegalovirus ou le virus syncytiel respiratoire.

4 - Procédé de congélation de cellules dans un récipient support consistant notamment dans des plaques multipuits ou des tubes permettant la conservation de cellules et leur utilisation après décongélation notamment dans un test de diagnostic selon l'une des revendications 1 à 3, caractérisé en ce que

1) on utilise un oryopréservant choisi de sorte que le nombre de cellules viables et fonctionnelles après décongélation soit optimal et notamment supérieur à 80 %, et

2) le nombre de cellules à congeler introduites dans le récipient support est choisi de manière à ce que 24 heures après décongélation les cellules cultivées forment une monocouche cellulaire complète.

5 - Procédé selon la revendication 4 caractérisé en ce que le récipient support en matière plastique est traité par un agent de revêtement choisi notamment parmi le collagène, la fibronectine, la polylysine, la concanavaline et éventuellement des mélanges de ceux-ci, notamment un mélange de collagène et fibronectine.

6 - Procédé selon l'une des revendications 4 ou 5, caractérisé en ce que l'agent cryopréservant est choisi parmi le DMSO, le glycérol, le tréhalose, le diméthylacétamide, le polyéthylèneglycérol, le polyvinyl pyrrolidone, dilué dans le milieu de culture seul ou de préférence additionné de sérum de veau foetal notamment 5 à 10 %, ou de sérum albumine notamment 2 à 5 %, ou du sérum de veau foetal pur.

7 - Procédé selon l'une des revendications 1 à 6, caractérisé en ce qu'on effectue la congélation de cellules en suspension dans un récipient pouvant être le récipient support.

8 - Procédé selon l'une des revendications 1 à 6, caractérisé en ce qu'on effectue la congélation de cellules en culture monocouche dans le récipient support.

9 - Procédé selon l'une des revendications 4 à 8, caractérisé en ce que les cellules sont des cellules embryonnaires de poumon humain de type fibroplastique MRC-5.

10 - Procédé selon l'une des revendications 4 à 8, caractérisé en ce que les cellules sont des cellules de rein de singe de type fibroplastique VERO.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,Y | JOURNAL OF CLINICAL MICROBIOLOGY vol. 19, no. 5, mai 1984, pages 631-633, Washington DC, USA; L. S. NERURKAR et al.: "Comparison of Standard Tissue Culture, Tissue Culture Plus Staining, and Direct Staining for Detection of Genital Herpes Simplex Virus Infection" * le document en entier * | 1 | C 12 N    1/04<br>G 01 N   33/569<br>G 01 N   33/571<br>C 12 N    5/00 |
| A | idem | 2,3 | |
| Y | FR-A-2 600 671   (INSTITUT MERIEUX) * revendications 1-14 * | 1 | |
| A | | 4-7,9, 10 | |
| A | EP-A-0 246 824   (CELL SYSTEMS LTD.) * le document en entier * | 4-7 | |
| A | CH-A-  439 593   (UNION CARBIDE CORPORATION) * page 9, lignes 40-60; revendication, sous-revendications 1-7 * | 4-8 | **DOMAINES TECHNIQUES RECHERCHES (Int. Cl.4)** |
| A | BIOPASCAL abrégé no. 73221374, 1973; M. J. ASHWOOD-SMITH et al.: "Low-temperature preservation of nammalian cells in tissue culture with polyvinylpyrrolidone (PVP), dextrans, and hydroxyethyl starch (HES)" & CRYOBIOLOGY, 1972, 9(5), 441-449 | 4,6 | C 12 N    1/00<br>A 01 N    1/00<br>C 12 Q    1/00<br>G 01 N   33/00 |

Le présent rapport a été établi pour toutes les revendications

| Lieu de la recherche | Date d'achèvement de la recherche | Examinateur |
|---|---|---|
| BERLIN | 12-07-1989 | DE KOK A.J. |

EPO FORM 1503 03.82 (P0402)